# EUROPEAN PATENT SPECIFICATION

(11) **EP 2 457 576 B1**
(45) Date of publication and mention of the grant of the patent: **08.07.2020**
(21) Application number: 10460047.3
(22) Date of filing: 29.11.2010
(51) Int. Cl.: A61K 35/74, C12N 1/20, C12R 1/225, A23L 33/10, A61P 3/04, A61P 3/10, A61P 3/06, A61P 9/12

(54) **Lactobacillus reuteri strain for use in the medical and veterinary prophylaxis and treatment of metabolic syndrome or infections**
Lactobacillus reuteri Stamm zur Verwendung der medizinischen und tiermedizinischen Prophylaxe und Behandlung von dem metabolischen Syndrom oder Infektionen
Lactobacillus reuteri pour l'utilisation dans la prophylaxie et le traitement médical et vétérinaire du syndrome métabolique ou des infections.

(43) Date of publication of application: 30.05.2012
(73) Proprietor: Eurochit Danuta Kruszewska, 02-765 Warszawa (PL)
(72) Inventor: Kruszewska, Danuta, 02-765 Warszawa (PL)
(74) Representative: Malewska, Ewa

(56) References cited:
- EP-A1- 2 221 359
- GERALD FALASCA ET AL: "New and improved strategies for the treatment of gout", INTERNATIONAL JOURNAL OF NEPHROLOGY AND RENOVASCULAR DISEASE, 24 November 2010 (2010-11-24), page 145, XP055035823, DOI: 10.2147/IJNRD.S6048

## Description

The present invention relates to a new use of a new nanoproduct useful in prophylaxis and medicine, both human and veterinary. The source of this nanoproduct is a new microorganisms isolated and identified by the inventor of the present invention - Danuta Kruszewska. This microorganism is capable of releasing products exhibiting activity useful in the prophylaxis and in human and veterinary medicine.

Throughout the lifetime of a healthy human (as well as other animals), microorganisms are present in his/her organism. Among these microorganisms, bacteria prevail, although fungi and protozoa are also present. Various types of microorganisms colonize most intensively the mucosa of the gastrointestinal tract, body surface and other systems, e.g. urogenital or upper airways, and settle on mucosal surfaces. The mechanisms, according to which individual species/strains of a specific microbiota, as well as the microbiota as a whole perform their function in the maintenance of health, have not been fully recognized. Microbiota of the gastrointestinal tract, the skin, and other ecological niches inhabited by microbes differ with respect to the functions performed, composition and amount of microorganisms. Thus, the term 'microbiota' is to be understood as referring to a 'herd' of microorganisms inhabiting in a specific anatomical and physiological region of the body, which constitutes a microbiom of the microorganisms.

Mutual interactions of the gastrointestinal tract microorganisms have the character of a symbiotic co-existence. Based on the example of the alimentary system, the microbiota of the system stimulate host immunity, not only on the level of a microorganism (live or dead), but also through its intra- and extra-cellular metabolites, counteract the anchoring of other microorganisms dangerous for health (mainly pathogens) - through competitiveness with respect to receptor and substrate, neutralize toxic intra- and extra-cellular metabolites of other microbes, regulate the development and physiological functions of the intestine (not only on the level of digestive functions), ferment indigestible, but energetically useful substrates, metabolize glycans and amino acids, and synthetize vitamins.

KR20030064462 discloses (cf. abstract) that *Lactobacillus pentosus K34* inhibits the growth of harmful bacterial selected from a group consisting of *Salmonella gallinarum, S. aureus, H. pylori, Lactobacillus rhamnosus, S. cerevisiae, E. coli, Phizopus stolonifer, Staphylococcus aureus* and *Propionic bacterium acnes.*

Lysozyme, a hydrolytic enzyme released by certain phagocytes, such as macrophages and multinuclear leukocytes, plays a significant role in the control of pathogenic microorganisms. Lysozyme is also produced by Paneth cells located in the lining of the intestines. Lysozyme is especially active against Gram-positive microorganisms. Phagocytic activity of cells involves degradation, with the participation of lysozyme, of the cellular walls of bacteria, more precisely - a cleavage of glycoside bonds in peptidoglycans.

Despite the possibilities of isolation and full identification of lysozyme, the therapeutic use of this compound is not possible due to its high activity. In the publication No. WO 89/11294 a possibility of the therapeutic use of the dimerized form of lysozyme is disclosed indicating its effectiveness in therapeutic use in human and veterinary medicine.

It is the aim of the invention to provide an agent useful in the prophylaxis and therapy of diseases of metabolic syndrome, ensuring an improvement in salubrity of the populations of highly developed countries, and eliminating social and economic costs associated with the prevalence of these diseases. Further aim of the invention is to provide an agent enhancing activity of the immune system of the body, thus increasing immunity minimize diseases and side effects induced by pathogenic microorganisms, especially by viruses and bacteria.

It has been unexpectedly found that the above-mentioned and other goals were achieved due to the development of a solution based on the isolation and identification of a new microorganism - *L. reuteri* DAN080, deposited on June 20, 2003 - in accordance with a Budapest Treaty on the International Recognition of the Deposit of Microorganisms for the Purposes of Patent Procedure, in DSMZ collection - Deutsche Sammlung von Mikroorganismen und Zellkulturen GmbH, in Braunschweig, DE, access number: DSM 15693.

The invention relates to a preparation in the form of the culture of *L. reuteri* DAN080, the partially inactivated culture of *L. reuteri* DAN080, liquid supernatant, concentrated supernatant and dried or lyophilized supernatant obtained from the culture of *L. reuteri* DAN080, as a therapeutic and/or prophylactic agent for for use in the treatment and prevention of diseases of metabolic syndrome or of infections by increasing the activity of lysozyme in a body of vertebrate, especially human, other mammal and bird.

Also, the whole culture of *L. reuteri* DAN080, liquid supernatant, concentrated supernatant, and dried or lyophilized supernatant obtained from the culture *of L. reuteri* DAN080, and from mixed bacterial cultures comprising *L. reuteri* DAN080, and liquid supernatants, concentrated supernatants and dried or lyophilized supernatants obtained from a *L. reuteri* DAN080 strain for use in the treatment or preventing the development of diseases of metabolic syndrome or of infections by increasing lysozyme activity in an organism of a vertebrate, especially human, other mammal or bird.

The invention relates also to the use of the above-mentioned agent for the manufacturing of a composition for the treatment and prevention of the development of diseases of metabolic syndrome or of infections by increasing the activity of lysozyme in an organism of a vertebrate, especially human, other mammal or bird.

The composition is intended for administration in an effective amount and at a suitable rate, indispensable for obtaining the desired preventive or therapeutic effect.

According to the invention, the composition is a pharmaceutical composition, optionally comprising other biologically active substances and vitamins D and E, especially in form of nanoparticles, in preventive or therapeutic doses, as well as pharmaceutically allowable carriers and/or additions.

Preferably, the pharmaceutical composition is in a solid form and is divided into single doses comprising therapeutically effective amount of the present therapeutic and/or preventive agent, in the amount of from 0.001 to 0.2 g/kg of body weight per day.

The pharmaceutical composition has in particular a form of a tablet or capsule.

In other embodiment, the pharmaceutical composition is in a liquid form and is divided into single doses comprising a therapeutically effective amount of the present therapeutic and/or preventive agent, in the amount of from 0.001 to 0.2 g/kg of body weight per day, especially in an ampoule.

The composition according to the invention is a dietary supplement, food or beverage, and optionally contains other biologically active substances and vitamins D and E, especially in the form of nanoparticles, in preventive doses.

Preferably, food additive, the food is in a solid form and/or in the form of a beverage.

According to the invention, the preventively effective amount is from 0.001 to 0.2 g/kg of body weight per day.

In further embodiment, the composition is feed or an additive to feed or beverage, and optionally contains other biologically active substances and vitamins D and E, especially in the form of nanoparticles, in preventive doses.

Preferably, feed or feed additive is in solid form and/or in the form of a beverage, and the preventively effective amount is from 0.001 to 0.2 g/kg of body weight perday.

The solution according to the invention has a stimulatory effect on the immune system, especially by increasing lysozyme activity and enhancing its antimicrobial and antiviral activity.

The invention is disclosed in details in the following detail description, with references being made to the accompanying drawing in which:

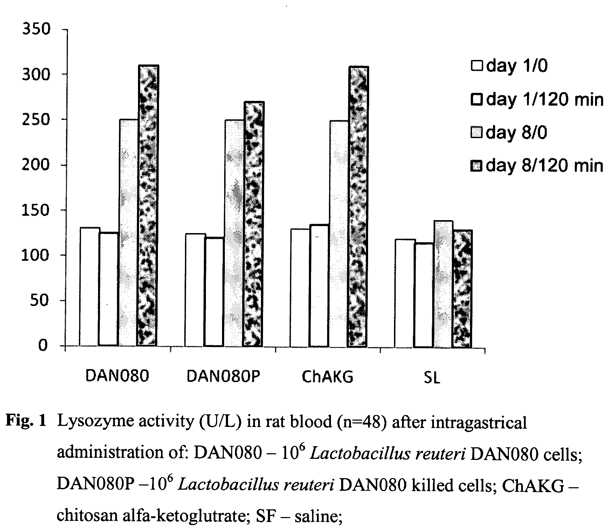

- Fig. 1: illustrates the study of lysozyme activity (U/L) in rat blood following intragastric administration *of L. reuteri* DAN080

### Detailed description of the invention

Bacteria *L. reuteri* DAN080 were isolated from the gastrointestinal tract of a healthy laboratory animal. The bacteria were isolated as a single colony on a solid medium with blood agar. This medium was incubated with the scrapings from the gastrointestinal tract of a healthy mouse at the temperature of 37°C for 24 hours. The colony isolated was proliferated in the broth MRSB (Oxoid), on a standard medium for lactic acid bacteria (LAB). The pH of the medium prior to sterilization was pH 6.8. Sterilization was performed within 15 min., at temp. of 121°C, the pH of the medium after sterilization: pH 6.2. The thermal conditions of bacteria incubation remained within the range 35±3°C. The full growth in the liquid culture was 16 hours. The bacteria can be stored, with warranty of survival at the temperature of -20°C., *L. reuteri* DAN080, survive at room temperature 20 to 22°C for at least 30 min. maintaining the capability of inhibiting the growth of other microorganisms.

In order to obtain an enhanced antimicrobial activity the bacteria *L. reuteri* DAN080 are grown on media comprising AKG.

Composition of the medium: 0.5% meat extract ; 0.5% yeast extract; 1% peptone; 0.3 % NH₄Cl; 0.4 % K₂HPO₄; 0.4 % KH₂PO₄; 0.01% MgSO₄ x 7 H₂O; 0.005 % MnSO₄ x 4 H₂O; 0.1% Tween 80; 0,05 L-cysteine HCl; 0.0002 of each of the following vitamins: B1, B2, B6, B5, B12, B9. After sterilization in the conditions as presented above, 23 mmol/l maltose and, respectively, starch or glucose, and 10 mmol/l AKG were added to the medium. The medium pH was: pH 6.2. The bacteria were incubated at 37°C for 16 hours. An enhanced bacterial growth occured in the presence of AKG. In the medium, from 4 to 6 mmol/l of acetates and lactates were found, while in the medium non-enriched with AKG - from 1 to 2 mmol/l.

*L. reuteri* DAN080 was identified in accordance with biochemical activity, where the capability of fermenting carbohydrates was assessed by the test - Api 50 CH and CHL medium, bioMerieux SA, Marcy 1Étoile, France.
Taxon: *L. reuteri*
- phenotypic characteristics according to the API system
   CAT: 1053 1121 0000 000 000 0000000
   API RID32s: 515 151 511 111 315 111 111 511 111 111 11 1
   API 50CHL: 1111533111 5511111111 1411111155 5511151111 1111111311
   Api ID32AN: 155 515 111 114 111 513 351 351111 312

Genotypic characteristics [SEQ. ID NO: 1, 2, 3]: based on DNA analysis and comparison with 16S gene sequence of rhibosomal RNA *L. reuteri* (see: GenBank: EF187261.2; Byun R, Nadkarni MA, Chhour KL, Martin FE, Jacques NA, Hunter N. Quantitative analysis of diverse Lactobacillus species present in advanced dental caries. J Clin Microbiol. 2004;42(7):3128-36; Fredricks DN, Relman DA. Improved amplification of microbial DNA from blood cultures by removal of the PCR inhibitor sodium polyanetholesulfonate. J Clin Microbiol. 1998; 36(10):2810-6). The following primers were useful for sequencing [SEQ. ID NO: 2, 3] - primer 1: TGGAAACAGA TGCTAATACC GC (22 bp) [SEQ. ID NO: 2], primer 2: ATTAGATACC CTGGTAGTCC (20 bp) [SEQ. ID NO: 3]

tggaaacaga tgctaatacc gcataacaac aaaagccaca tggcttttgt
ttgaaagatg gctttagcta tcactctggg atggacctgc ggtgcattag
ctagttggta aggtaacggc ttaccaaggc gatgatgcat agccgagttg
agagactgat cggccacaat ggaactgaga cacggtccat actcctacgg
gaggcagcag tagggaatct tccacaatgg gcgcaagcct gatggagcaa
caccgcgtga gtgaagaagg gtttcggctc gtaaagctct gttgttggag
aagaacgtgc gtgagagtaa ctgttcacgc agtgacggta tccaaccaga
aagtcacggc taactacgtg ccagcagccg cggtaatacg taggtggcaa
gcgttatccg gatttattgg gcgtaaagcg agcgcaggcg gttgcttagg
tctgatgtga aagccttcgg cttaaccgaa gaagtgcatc ggaaaccggg
ccacttgagt gcagaagagg acagtggaac tccatgtgta gcggtggaat
gcgtagatat atggaagaac accagtggcg aaggcggctg tctggtctgc
aactgacgct gaggctcgaa agcatgggta gcgaacagga ttagataccc
tggtagtcc [SEQ. ID NO: 1] (659 bp)

After a specified time *of L. reuteri* DAN080 growth, the culture is centrifuged and the liquid supernatant, concentrated supernatant, and dried or lyophilized supernatant is the product of a specific capability and activity for regulating the growth of *H. pylori* and other bacteria *in vitro* and *in vivo.* After electrophoretic separation performed from the liquid supernatant, the concentrated supernatant and dried supernatant collected at a predetermined time, specific proteins of a molecular weight within the range 150 - 22 kD or less were visualized, which proteins are responsible for homeostasis and regulation of the growth of *H. pylori.* These bands are weak, and do not occur in electrophoregraphs obtained from liquid supernatant, concentrated supernatant and dried supernatant harvested at a time different than the above-specified time for *L. reuteri* DAN080 culture, and the liquid supernatant, concentrated supernatant and dried supernatant, show one of the proper effects - homeostatic and growth regulating, on *H. pylori* and other bacteria *in vitro,* as well as *in vivo.*

For the genetic identification of *L. reuteri,* the total genomic DNA was isolated from bacteria cultured overnight, with the use of the kits DNaesy™, Qiagen GmbH, Hilden, Germany.

Amplification of the 340 bp fragment of 16S rDNA was performed with semi-nested PCR (first run: 94°C for 30 s., 61°C for 60 s., 68°C for 60 s., 35 cycles; second run: 94°C for 30 s., 58°C for 60 s., 68°C for 60 s., 40 cycles) using primers [SEQ. ID NO: 4, 5, 6] (see: Walter, J., Hertel, Ch., Tannock GW., Lis CM., Munro K., Hammes W.P. (2001) Detection of Lactobacillus, Pediococcus, Leuconostoc i Weisella species in human faeces by using group specific PCR primers and denaturing gradient gel electrophoresis. Appl. Environ. Microb. 67, 2578-2585); forward primer 3: AGCAGTAGGG AATCTTCCA (19 bp) [SEQ. ID NO: 4]; reverse primer 4: ATTYCACCGC TACACATG (18 bp) [SEQ. ID NO: 5]; forward primer 5: ACAATGGACG AAAGTCTGAG TG (22 bp) [SEQ. ID NO: 6].

At present, it has been found that the changes of nutritional habits and the accompanying enzymatic deficiency on the level of gastrointestinal tract (enzymes) are accompanied by unexpected processes of regrouping of natural microbiota, with respect to the composition and amount of the autochtonic inhabitant microorganisms. In place of the natural, innate microbiota of a healthy host, or a host with no relapse of gout attack the mucosal surfaces are colonized by other bacterial species, with various intensity, different from the natural. These 'new' microorganisms, while settling new ecological niches, revealtheir own virulence factors. Then, according to the degree of virulence, a local/systemic infection and local or systemic inflammation develop. These processes take place mainly in the gastrointestinal tract and urogenital system.

*Role of vitamin D:* In gout patients, the blood level of 1.25(OH)2-vitamin D3 is significantly lower (p < 0.05), when compared to the concentration of this vitamin in healthy individuals (8.8 mg/dL +/- 0.2 vs. 5.6 +/- 0.2 mg/dL), whereas among males suffering from gout and those free from podagra no differences are observed in the level 25(OH)-vitamin D3. It is thus clear, that in gout patients, uric acid *per se* may directly reduce the level of 1.25(OH)2-vitamin D3 in blood by inhibiting 1-hydrolase activity (see: Takahashi S, Yamamoto T, Moriwaki Y, Tsutsumi Z, Yamakita J, Higashino K. Decreased serum concentrations of 1,25(OH)2-vitamin D3 in patients with gout. Adv Exp Med Biol. 1998;431:57-60).

The role of vitamin D and its active metabolites in the defense responses of the organism covers several levels.

On the first level are epithelial cells which constitute a physical barrier protecting against injury and/or infection/invasion. Active hormone 1.25(OH)2 vitamin D enhances the physical barrier by stimulating genes encoding gap junction proteins, adherence genes, tight junction genes, and enhances intercellular communication (proteins: connexin 43, E-cadherin, occludin).

Secondly, vitamin D has a stimulatory effect on epithelial cells in the synthesis of antimicrobial peptides of innate immunity, including beta-defensins, cathelicidin LL-37.

Subsequently, vitamin D stimulates expression of potentially active antimicrobial peptides synthetized in macrophages/neutrophils, and increases the potential of oxygen burst in macrophages.

Besides, it enhances the neutralization of endotoxins through LL-37.

With respect to acquired immunity, vitamin D shows a suppressive effect, manifested as its capability for the inhibition of T lymphocytes proliferation. It exerts a suppressive effect on immunity dependent on the production of cytokines and immunoglobulins through activated B lymphocytes. It inhibits the activity of Th1 lymphocytes, and reduces synthesis by Th1 IF-gamma and IL-2 (stimulator of antibodies and cytokines). These lymphocytes participate in the development of disorders of the autoimmune background (e.g. type 1 diabetes, rheumatoid arthritis, autoimmune inflammation of the intestines, and multiple sclerosis).

*The role of vitamin E:* A relationship was shown between a diet, and more precisely between higher consumption of meat and total proteins and decreased consumption of fruits, vegetables and vitamin C, and a risk of gout development. It was confirmed earlier that red meat, fruits of the sea, beer and high proof alcohol, and also total protein, wine and vegetables rich in purines increase a risk of gout development; while recently, dairy products have been identified as protective agents. Clinical studies in humans showed that vitamins of anti-oxidative activity (vitamin E, vitamin C, beta-carotene, vitamin A) do not significantly inhibit the process of osteoarticular inflammation of the knee, as has been previously suggested. However, in view of the fact that diet is inevitably commonly available, it is considered that even a slight improvement of health, which is the result of nutritional changes, may lead to a great effect on the health of the population. Since there is evidence showing the effect of nutritional factors on, among other factors, the course of podagra, they should not be belittled, but commonly popularized (see: Choi HK. Dietary risk factors for rheumatic diseases. Curr Opin Rheumatol. 2005;17(2):141-6).

In accordance with the present invention, vitamins D and E are used in the form of commercially available nanoparticles. Nanoparticles of vitamins D and E are characterized by higher bioavailability. However, following an oral administration, the measurements of the levels of these vitamins in blood, performed within the standard periods after administration show a normal or only slightly elevated values. The measurement performed within a time shorter than the standard ones confirms an elevated bioavailability of vitamins D and E.

It is known that in the course of gout, the intestinal tract enzymes decomposing food rich with proteins and purine compounds do not function properly.

In the application No. WO 1988/008450 - "Gene therapy for metabolite disorders", it is only shown that a new therapy is possible, the therapy being based on recombinants for the treatment and prevention of undesired conditions characterized by accumulation or increased concentration of metabolites. The inventors of the solution propose the use of recombinants producing oxalate oxidase and oxalate decarboxylase in order to prevent oxalic acid diathesis and the formation of kidney stones. Apart from this, a recombinant with gene encoding uricase is to be useful for metabolisation of uric acid. Such a therapy is to treat and prevent gout and formation of stones. *Oxalobacter formigenes* OxB (ATCC 35274) - a bacterium naturally settling in the human gastrointestinal tract, is the microorganism, being a source of the genes encoding oxalate decarboxylase and oxalate oxidase inserted into the recombinants. Uricase gene was isolated from pig's liver.

*Uric acid metabolism:* In primates, birds and some reptiles, uric acid is the final product of purine metabolism. In the human body adenine and guanine are metabolised to xanthine. From xanthine, in turn, after oxidation with xanthine oxidase, uric acid is formed according to the reaction:

xanthine + H O + 0_ - > uric acid + 0 ∼

Superoxide dismutase converts superoxide anion radical (O ∼) to hydrogen peroxide (Lehninger, A.L: 1975, Biochemistry, 2nd Edition., Worth Publishers, New York, pp. 740 - 741). Enzymes participating in the metabolism of uric acid commonly occur among mammals, excluding humans. In these animals, urates are re-adsorbed in kidney and transported to liver where, with the participation of hepatic uricase, urates are converted to alantoin soluble in water, whereas humans are genetically predisposed to the formation of kidney stones (see: Gutman AB, Yu T-F: Uric acid nepholithiasis, 1968, Am. J. Med. 45:756- 779).

In accordance with the present invention, the anti-microbial agent selected from the group comprising the whole culture *of L. reuteri* DAN080, liquid supernatant, concentrated supernatant and dried or lyophilized supernatant obtained from the culture *of L. reuteri* DAN080, and from mixed bacterial cultures comprising *L. reuteri* DAN080, and liquid supernatants, concentrated supernatants and dried or lyophilized supernatants obtained from *L. reuteri* DAN08 is for use in prophylaxis and treatment of medical conditions developing as a result of infections caused by bacteria, fungi and other pathogens of the gastrointestinal tract, body integuments and other systems, such as the urogenital and respiratory systems in vertebrates.

Similar to other probiotics, the probiotic potential of the bacteria *L. reuteri* DAN080 is evaluated based on the possibilities of passage through the gastrointestinal tract, production of antimicrobial compounds, degree of adherence to the epithelial mucin (e.g. intestinal epithelium), production of biogenic amines, mucin degradation, drug sensitivity pattern.

During fermentation, which takes place in the gastrointestinal tract, urogenital tract, body cavities and ducts, considerable amounts of final acidic metabolites are released by bacteria, accompanied by a decrease in pH. The products are difficult to quantify and include hydrogen peroxide and diacetyl, being the agents regulating microbiological relationships in the environment (antibiosis). Bacteriocins are important in the selection of microbiota triggering the fermentation. The strain/strains were identified and characterized morphologically and biochemically, as well as molecularly (identification), with respect to capabilities of survival in an acidic environment, in the presence of bile salts, capability for the utilization of proteins, starch, fats, for production of hydrogen peroxide, for bile salts hydrolase activity, and also for the production of substances inhibiting the growth of other bacteria undesirable in the gastrointestinal tract, and the determination of resistance to antimicrobial compounds. The evaluation shows non-infectiousness of the strain *L. reuteri* DAN080, which was tested on animals with impaired immunity.

Gram-positive bacteria encode proteins required for the incorporation into own cellular wall (D-alanine esters). This process, with the participation of teichoic acids, is important for the bacterial cell and its tolerance to acidic character of the environment, its resistance to antimicrobial peptides, its adhesion, formation of a biofilm, and degree of its virulence. The presence of D-alanine residues is important for the functioning *of L. reuteri* DAN080 cells and their survival in the gastrointestinal tract. It was found that treatment with catalase, changes of pH, and heating up to 80°C do not affect the bactericidal activity *of L. reuteri* DAN080. Even the treatment with trypsin and proteinase K did not affect this characteristic. No reduction in antimicrobial activity was observed when an increased availability of glucose (source of carbon) and peptone (source of nitrogen) in the medium has occurred. The bacteria *L. reuteri* DAN080 survived also in pH 3 and subsequently were not sensitive to the activity of cholic acid and bovine bile, while still exhibiting bile salts hydrolase activity, and ability to produce antimicrobial compounds.

*L. reuteri* (LR) - including *L. reuteri* DAN080, are microorganisms which may produce hazardous primary and secondary metabolites, including organic acids, diacetyl, CO₂ and various antibiotic-like substances, such as reuterin, reutericin, reutericyclin, cobalamin, etc.

Some strains of LR possess a capability of synthetizing and releasing bacteriocins. One of these is reutericin 6 - a bacteriocin, which shows both bactericidal and bacterioststic activity with respect to many species of bacteria, especially those Gram-positive. Lytic power was the strongest in the poorly opaque environment with a small number of live cells, in presence of beta-galactosidase (leakage from bacterial cells). This bacteriocin is not active against Gram-negative bacteria and does not occur in the strains of *L. reuteri* producing reuterin.

Reutericin 6 has a molecular weight of 2.7 kDa and comprises 67% of hydrophobic and polarly neutral amino acids, among which no lanthionine is found. The structure of the molecule is cyclic, and impossible to differentiate from gasericin A (similar molecular weight and amino acid sequence). Both bacteriocins differ with respect to bactericidal strength. Although they cause leakage of potassium ions from the cell and from liposomes, the strength of the leakage is different. Structurally, the bacteriocins are mainly in the form of alpha helises, with the difference in the number of amino acids with D and L configuration present. Reutericin 6 has two D-alanine residues among all 18 alanine residues present, while in gasericin there is only one such a residue. The number of amino acids residues, differing in their D or L configuration, determines the bactericidal activity of LR.

LR show anti-microbial activity, for which none of the known bacteriocins or reuterin or else organic acids are responsible. Reutericyclin shows a wide inhibitory spectrum of antimicrobial activity. Its activity does not inhibit the growth of Gram-negative microorganisms; however, *E. coli* mutant, with the LPS structure different from that of the non-mutant strain, is sensitive to the effect of reutericyclin. Reutericyclin acts against cells in a dose-dependent way. It does not destroy spores, but violates the conditions in which the germination of spores occurs. The addition of fatty acids to the bacterial culture medium changes the activity of reutericyclin. Reutericyclin - as a molecule, is hydrophobic, has a negative charge, and molecular weight of 3.49 kDa. Structurally, reutericyclin is a derivative of tetram acid (see: A. Höltzel, M. G. Gänzle, G. J. Nicholson, W. P. Hammes, and G. Jung, Angew. Chem. Int. Ed. 39:2766-2768, 2000).

Reuterin production is enhanced in the presence of glycerol.

Reuterin is a substance of antimicrobial activity produced mainly by *L. reuteri* during the process of anaerobic fermentation in the presence of glycerol. Maximum production of this substance occurs in the static phase and the phase of logarithmic bacterial growth.

In the presence of glycerol, *L. reuteri* synthetizes β-hydroxypropanal (HPA) which is subsequently secreted into the medium. It was confirmed that in a water solution reuterin occurs as a mixture of three forms of β-hydroxypropanal: monomeric, hydrated and dimeric, which remain in balance.

This compound was first isolated, purified and identified by Talarico and Dobrogosz. To-date, a number of reuterin properties have been demonstrated, primarily it is an effective inhibitor of the growth of a wide spectrum of not only bacteria, but also of fungi and protozoa. The mechanism of reuterin activity has been investigated for over 20 years, and at present it is known that this compound may exert an effect on microorganisms in a dual manner. The substance may inhibit the activity of bacterial ribonucleotide reductase (an enzyme catalyzing the first stage of DNA synthesis) by competing with ribonucleotides for binding sites in the DNA sequence, or by reaction with unstable thiol groups of this enzyme. In addition, it was found that reuterin may enter into direct reactions with thioredoxin, a protein performing the role of a reducer of many enzymes, including ribonucleotide reductase, thus inhibiting the enzymatic activity of this protein. Reuterin is a substance soluble in water, acting within a wide pH values, resistant to lipolytic and proteolytic enzymes treatment. The optimum conditions for the growth and production of reuterin by *L. reuteri* is the temperature of 37°C and pH 4.6-5; this compound also remains stable in an environment of a considerably lower temperature and acidity of the medium.

The strains of LR also produce cobalamin (vitamin B12) in the process of cofermentation of glycerol and glucose.

*Genetics:* It is possible to construct a shuttle vector (e.g. *Escherichia coli-*lactobacillus) transferred into *L. reuteri* DAN080 cells, so that the transformant obtained showed its activity, e.g. antimicrobial.

There is a possibility of cloning genes in *L. reuteri* DAN080, (known is the cloning of beta-galactosidase heterodimer in *L. reuteri* cells other than *L. reuteri* DAN080 cells), and it is assumed that the expression of such structural genes must be associated with the activity of proteins involved in maturation (cutting, cyclic form) and secretion outside the *L. reuteri* DAN080 cell by various transporting systems. It is also assumed that in *L. reuteri* DAN080 the mechanisms significant for the autoprotection of cells against such strong inhibitors as e.g. reutericin 6, are present.

It is possible to construct with *L. reuteri* DAN080 cells a ligated gene of the green fluorescence protein into the secretion vector which generates release of a chimeric protein capable of glittering (marker).

It is possible to adapt *L. reuteri* DAN080 cells to be incorporated into the Nisin-controlled gene expression (NICE) system by ligating nisA promoter (PnisA) and nisRK DNA fragments into the shuttle vector *E. coli-L. reuteri* pSTE32. In such a chimeric plasmid the expression of heterologic genes is possible with the induction of nisin.

### EXAMPLES

### Example 1. The in vivo lysozyme activity test

Forty eight 2-month-old Sprague Dawley female rats with a weight of 140-275 g, were fed with feed adequate for the age of the animals, and watered *ad libitum.* Three days prior to experiment, all animals had catheters inserted in the jugular vein. The study was started by taking blood samples from the rats. Subsequently, the animals were administered intragastrically, by means of a gastric tube, 0.5 ml of the following preparations: suspension of the bacteria *L. reuteri* DAN080 - live and dead cells, chitosan AKG suspension, saline. Hundred twenty min. after the first blood taking the second blood sample was taken from the animals. On the second day, the rats received the same preparations once daily for 7 subsequent days. Twelve rats received live bacteria at a dose of 10⁶ cells suspended in physiological saline. The following 12 animals also received for 8 days, 10⁶ each of thermally killed cells *L. reuteri* DAN080. To the next 12 rats chitosan AKG suspension was administered, and the fourth group of animals (n=12) were administered intragastrically for 8 days, at each time 0.5 ml physiological saline. On day 8 after the final dose of the preparations administered intragastrically, blood was taken from the animals from the jugular vein. For the second time on the same day, blood was taken from all rats 120 min. after the first blood taking.

Determinations of the lysozyme activity in the blood were performed in the presence of a suspension of *Micrococcus lysodeikticus* cells of specified density, based on the absorbance value, and comparing this value with the absorbance curve plotted from a number of standard dilutions of crystal lysozyme (Sigma-Aldrich) in PBS and suspension of *M. lysodeikticus* cells of specified density. After 15, 30, 45, 60 min., incubation absorbance was measured at the wave length of 540 nm.

Results: A stimulation of rat immune system was observed by live and thermally killed bacteria *L. reuteri* DAN080 and by chitosan AKG. Fig. 1 presents the results.

### SEQUENCE LISTING

<110> EUROCHIT Danuta KRUSZEWSKA,
<120> NEW USE OF NEW NANOPRODUCT USEFUL IN PROPHILAXIS AND IN MEDICAL AND VETERINARY TREATMENT
   (Nowe zastosowanie nowego nanoproduktu uzytecznego w profilaktyce i medycynie ludzkiej oraz weterynaryjnej)
<130> I/171700.EP
<140> not known yet
   <141> 2010-11-29
<160> 6
<170> PatentIn version 3.3
<210> 1
   <211> 659
   <212> DNA
   <213> murine
<400> 1 
tggaaacaga tgctaatacc gcataacaac aaaagccaca tggcttttgt
ttgaaagatg gctttagcta tcactctggg atggacctgc ggtgcattag 100
ctagttggta aggtaacggc ttaccaaggc gatgatgcat agccgagttg
agagactgat cggccacaat ggaactgaga cacggtccat actcctacgg 200
gaggcagcag tagggaatct tccacaatgg gcgcaagcct gatggagcaa
caccgcgtga gtgaagaagg gtttcggctc gtaaagctct gttgttggag 300
aagaacgtgc gtgagagtaa ctgttcacgc agtgacggta tccaaccaga
aagtcacggc taactacgtg ccagcagccg cggtaatacg taggtggcaa 400
gcgttatccg gatttattgg gcgtaaagcg agcgcaggcg gttgcttagg
tctgatgtga aagccttcgg cttaaccgaa gaagtgcatc ggaaaccggg 500
ccacttgagt gcagaagagg acagtggaac tccatgtgta gcggtggaat
gcgtagatat atggaagaac accagtggcg aaggcggctg tctggtctgc 600
aactgacgct gaggctcgaa agcatgggta gcgaacagga ttagataccc
tggtagtcc 659
<210> 2
   <211> 22
   <212> primer 1
<400> 2
   TGGAAACAGA TGCTAATACC GC 22
   Reference: Left primer (genus specific): LactoF (Byun R, Nadkarni MA, chhour KL, Martin FE, Jacques NA, Hunter N. Quantitative analysis of diverse Lactobacillus species present in advanced dental caries.
   J Clin Microbiol. 2004;42(7):3128-36).
<210> 3
   <211> 20
   <212> primer 2
<400> 3
   ATTAGATACC CTGGTAGTCC 20
   Reference: Right primer 806F (Fredricks DN, Relman DA.

### SEQUENCE LISTING

Improved amplification of microbial DNA from blood cultures by removal of the PCR inhibitor sodium polyanetholesulfonate.
J Clin Microbiol. 1998; 36(10):2810-6).
<210> 4
   <211> 19
   <212> primer 3
<400> 4
   AGCAGTAGGG AATCTTCCA 19
<210> 5
   <211> 18
   <212> primer 4
<400> 5
   ATTYCACCGC TACACATG 18
<210> 6
   <211> 22
   <212> primer 5
<400> 6
   ACAATGGACG AAAGTCTGAG TG 22

Reference for primers 3-5 (seg.4-6): Walter, J., Hertel, Ch., Tannock GW., Lis CM., Munro K., Hammes W.P. (2001) Detection of Lactobacillus, Pediococcus, Leuconostoc i weisella species in human feces by using group specific PCR primers and denaturing gradient gel elektophoresis. Appl. Environ. Microb. 67, 2578-2585

## Claims

1. A preparation in a form of a *Lactobacillus reuteri* DAN080 strain with deposit number DSM 15693 culture, a partially inactivated *L. reuteri* DAN080 strain with deposit number DSM 15693 culture, liquid supernatant, concentrated supernatant and dried or lyophilized supernatant obtained from *a L. reuteri* DAN080 strain with deposit number DSM 15693 culture, for use in a method for prophylaxis or treatment of diseases of metabolic syndrome by increasing the activity of lysozyme in an organism of vertebrate, especially human, other mammal or bird, the vertebrate being in need of such a treatment.

2. A preparation in a form of a whole *L. reuteri* DAN080 strain with deposit number DSM 15693 culture, liquid supernatant, concentrated supernatant, and dried or lyophilized supernatant obtained from a *L. reuteri* DAN080 strain with deposit number DSM 15693 culture, and from mixed bacterial cultures comprising *L. reuteri* DAN080 strain with deposit number DSM 15693, for use in a method for prophylaxis or treatment of diseases of metabolic syndrome by increasing lysozyme activity in an organism of a vertebrate, especially human, other mammal or bird, the vertebrate being in need of such a treatment.

3. Use of the preparation as defined in claims 1-2, for manufacturing of a composition for prophylaxis or treatment of diseases of metabolic syndrome by increasing lysozyme activity in an organism of a vertebrate, especially human, other mammal or bird, the vertebrate being in need of such a treatment.

4. A preparation in a form of a *Lactobacillus reuteri* DAN080 strain with deposit number DSM 15693 culture, a partially inactivated *L. reuteri* DAN080 strain with deposit number DSM 15693 culture, liquid supernatant, concentrated supernatant and dried or lyophilized supernatant obtained from a *L. reuteri* DAN080 strain with deposit number DSM 15693 culture, for use in a method for enhancing activity of the immune system in prophylaxis or treatment of infections by increasing the activity of lysozyme in an organism of vertebrate, especially human, other mammal or bird, the vertebrate being in need of such a treatment.

5. A preparation in a form of a whole *L. reuteri* DAN080 strain with deposit number DSM 15693 culture, liquid supernatant, concentrated supernatant, and dried or lyophilized supernatant obtained from a *L. reuteri* DAN080 strain with deposit number DSM 15693 culture, and from mixed bacterial cultures comprising *L. reuteri* DAN080 strain with deposit number DSM 15693, for use in a method for enhancing activity of the immune system in prophylaxis or treatment of infections by increasing lysozyme activity in an organism of a vertebrate, especially human, other mammal or bird, the vertebrate being in need of such a treatment.

6. Use of the preparation as defined in claims 4-5, for manufacturing of a composition for prophylaxis or treatment of infections by increasing lysozyme activity in an organism of a vertebrate, especially human, other mammal or bird, the vertebrate being in need of such a treatment.

7. The use according to claim 3 or 6, wherein the preparation is intended for administration in an effective amount and at a suitable rate, indispensable for obtaining the desired effect.

8. The use according to claims 7, wherein the preparation is a pharmaceutical composition, optionally comprising other biologically active substances and vitamins D and E, especially in form of nanoparticles, as well as pharmaceutically acceptable carriers and/or additives.

9. The use according to claim 8, wherein the pharmaceutical composition is in solid form, preferably in form of a tablet or capsule and divided into single doses comprising an effective amount of said preparation, ranging from 0.001 to 0.2 g/kg of body weight per day.

10. The use according to claim 8, wherein the pharmaceutical composition is in liquid form, divided into single doses comprising an effective amount of said preparation, ranging from 0.001 to 0.2 g/kg of body weight per day, especially in an ampoule.

11. The use according to claims 3, 6-7, wherein the composition is a dietary supplement, food in a solid form and/or beverage, optionally comprising other biologically active substances and vitamins D and E, especially in the form of nanoparticles.

12. The use according to claims 11, wherein the effective amount ranges from 0.001 to 0.2 g/kg of body weight per day.

13. The use according to claims 3, 6-7, wherein the composition is a feed or an additive to a feed in solid form and/or a beverage, optionally comprising other biologically active substances and vitamins D and E, especially in form of nanoparticles.

14. The use according to claims 13, wherein the effective amount ranges from 0.001 to 0.2 g/kg of body weight per day.

## Patentansprüche

1. Zubereitung in Form eines *Lactobacillus reuteri* DAN080 Stamm deponierte unter der Nummer DSM 15693-Kultur, eines teilweise inaktivierten *L. reuteri* DAN080 Stamm deponierte unter der Nummer DSM 15693-Kultur, flüssiger Überstand, konzentrierter Überstand und getrockneter oder lyophilisierter Überstand, erhalten aus einem *L. reuteri* DAN080 Stamm deponierte unter der Nummer DSM 15693, zur Verwendung in einem Verfahren zur Prophylaxe oder Behandlung von Erkrankungen des metabolischen Syndroms durch Erhöhung der Aktivität von Lysozym in einem Organismus von Wirbeltieren, insbesondere Menschen, anderen Säugetieren oder Vögeln, deren Wirbeltiere bedürfen eine solche Behandlung.

2. Zubereitung in Form eines ganzen *L. reuteri* DAN080 Stamm deponierte unter der Nummer DSM 15693-Kultur, flüssigem Überstand, konzentriertem Überstand und getrocknetem oder lyophilisiertem Überstand, erhalten aus einem *L. reuteri* DAN080 Stamm deponierte unter der Nummer DSM 15693-Kultur und aus gemischte Bakterienkulturen, die den Stamm *L*. *reuteri* DAN080 deponierte unter der Nummer DSM 15693 umfassen, zur Verwendung in einem Verfahren zur Prophylaxe oder Behandlung von Erkrankungen des metabolischen Syndroms durch Erhöhung der Lysozymaktivität in einem Organismus eines Wirbeltiers, insbesondere eines Menschen, eines anderen Säugetiers oder Vogels, des Wirbeltiers eine solche Behandlung benötigen.

3. Verwendung der Zubereitung nach Ansprüchen von 1 bis 2 zur Herstellung einer Zusammensetzung zur Prophylaxe oder Behandlung von Erkrankungen des metabolischen Syndroms durch Erhöhung der Lysozymaktivität in einem Organismus eines Wirbeltiers, insbesondere eines Menschen, eines anderen Säugetiers oder Vogels, wobei das Wirbeltier ist eine solche Behandlung benötigen.

4. Zubereitung in Form eines *L*. *reuteri* DAN080 Stamm deponierte unter der Nummer DSM 15693-Kultur, eines teilweise inaktivierten *L. reuteri* DAN080 Stamm deponierte unter der Nummer DSM 15693-Kultur, flüssiger Überstand, konzentrierter Überstand und getrockneter oder lyophilisierter Überstand, erhalten aus einem Stamm *L. reuteri* DAN080 deponierte unter der Nummer DSM 15693 zur Verwendung in einem Verfahren zur Steigerung der Aktivität des Immunsystems bei der Prophylaxe oder Behandlung von Infektionen durch Erhöhung der Aktivität von Lysozym in einem Organismus von Wirbeltieren, insbesondere Menschen, anderen Säugetieren oder Vögeln, Wirbeltieren eine solche Behandlung benötigen.

5. Zubereitung in Form eines ganzen *L. reuteri* DAN080 Stamm deponierte unter der Nummer DSM 15693-Kultur, flüssigem Überstand, konzentriertem Überstand und getrocknetem oder lyophilisiertem Überstand, erhalten aus einem *L. reuteri* DAN080 Stamm deponierte unter der Nummer DSM 15693-Kultur und aus gemischte Bakterienkulturen, die den Stamm *L. reuteri* DAN080 deponierte unter der Nummer DSM 15693 umfassen, zur Verwendung in einem Verfahren zur Steigerung der Aktivität des Immunsystems bei der Prophylaxe oder Behandlung von Infektionen durch Erhöhung der Lysozymaktivität in einem Organismus eines Wirbeltiers, insbesondere eines Menschen, eines anderen Säugers oder Vogel, wobei das Wirbeltier eine solche Behandlung benötigt

6. Verwendung der Zubereitung nach Ansprüchen von 4 bis 5 zur Herstellung einer Zusammensetzung zur Prophylaxe oder Behandlung von Infektionen durch Erhöhung der Lysozymaktivität in einem Organismus eines Wirbeltiers, insbesondere eines Menschen, eines anderen Säugetiers oder Vogels, wobei das Wirbeltier benötigt wird eine solche Behandlung.

7. Verwendung nach Anspruch 3 oder 6, wobei das Präparat zur Verabreichung in einer wirksamen Menge und mit einer geeigneten Geschwindigkeit vorgesehen ist, die zur Erzielung der gewünschten Wirkung unabdingbar ist.

8. Verwendung nach Anspruch 7, wobei die Zubereitung eine pharmazeutische Zusammensetzung ist, die gegebenenfalls andere biologisch aktive Substanzen und Vitamine D und E, insbesondere in Form von Nanopartikeln, sowie pharmazeutisch verträgliche Träger und / oder Zusatzstoffe enthält.

9. Verwendung nach Anspruch 8, wobei die pharmazeutische Zusammensetzung in fester Form vorliegt, vorzugsweise in Form einer Tablette oder Kapsel, und in Einzeldosen unterteilt ist, die eine wirksame Menge der Zubereitung im Bereich von 0,001 bis 0,2 g / kg Körpergewicht umfassen pro Tag.

10. Verwendung nach Anspruch 8, wobei die pharmazeutische Zusammensetzung in flüssiger Form vorliegt, aufgeteilt in Einzeldosen, die eine wirksame Menge der Zubereitung im Bereich von 0,001 bis 0,2 g / kg Körpergewicht pro Tag, insbesondere in einer Ampulle, umfassen.

11. Verwendung nach Ansprüchen 3, 6 bis 7, wobei die Zusammensetzung ein Nahrungsergänzungsmittel, Lebensmittel in fester Form und / oder Getränk ist, gegebenenfalls enthaltend andere biologisch aktive Substanzen und Vitamine D und E, insbesondere in Form von Nanopartikeln.

12. Verwendung nach Anspruch 11, wobei die wirksame Menge im Bereich von 0,001 bis 0,2 g / kg Körpergewicht pro Tag liegt.

13. Verwendung nach Ansprüchen 3, von 6 bis 7, wobei die Zusammensetzung ein Futter oder ein Zusatz zu einem Futter in fester Form und / oder einem Getränk ist, gegebenenfalls enthaltend andere biologisch aktive Substanzen und Vitamine D und E, insbesondere in Form von Nanopartikel.

14. Verwendung nach Anspruch 13, wobei die wirksame Menge im Bereich von 0,001 bis 0,2 g / kg Körpergewicht pro Tag liegt.

## Revendications

1. Une préparation sous forme de la souche de *Lactobacillus reuteri* DAN080 déposée sous le numéro DSM 15693, une souche *L. reuteri* DAN080 partiellement inactivée déposée sous le numéro DSM 15693, surnageant liquide, surnageant concentré et surnageant séché ou lyophilisé obtenu à partir d'un souche *L. reuteri* DAN080 avec culture déposée sous le numéro DSM 15693, destinée à être utilisée dans une méthode de prophylaxie ou de traitement des maladies du syndrome métabolique en augmentant l'activité du lysozyme dans un organisme de vertébrés, en particulier humain, autre mammifère ou oiseau, le vertébré ayant besoin de un tel traitement.

2. Une préparation sous la forme de la souche de *L. reuteri* DAN080 entière déposée sous le numéro DSM 15693, surnageant liquide, surnageant concentré et surnageant séché ou lyophilisé obtenu à partir de la souche de *L. reuteri* DAN080 déposée sous le numéro DSM 15693 culture, et de cultures bactériennes mixtes comprenant de la souche de *L. reuteri* DAN080 déposée sous le numéro DSM 15693, à utiliser dans une méthode de prophylaxie ou de traitement des maladies du syndrome métabolique en augmentant l'activité du lysozyme dans un organisme d'un vertébré, en particulier humain, autre mammifère ou oiseau, le vertébré ayant besoin d'un tel traitement.

3. Utilisation de la préparation telle que définie selon les revendications 1-2, pour la fabrication d'une composition pour la prophylaxie ou le traitement des maladies du syndrome métabolique en augmentant l'activité du lysozyme dans un organisme d'un vertébré, en particulier humain, autre mammifère ou oiseau, le vertébré étant besoin d'un tel traitement.

4. Une préparation sous forme de la souche de *L. reuteri* DAN080 déposée sous le numéro DSM 15693, une souche *L. reuteri* DAN080 partiellement inactivée déposée sous le numéro DSM 15693, surnageant liquide, surnageant concentré et surnageant séché ou lyophilisé obtenu à partir de la souche de *L. reuteri* DAN080 avec culture déposée sous le numéro DSM 15693, destinée à être utilisée dans une méthode pour améliorer l'activité du système immunitaire dans la prophylaxie ou le traitement des infections en augmentant l'activité du lysozyme dans un organisme de vertébrés, en particulier humain, autre mammifère ou oiseau, le vertébré ayant besoin d'un tel traitement.

5. Une préparation sous la forme de la souche de *L. reuteri* DAN080 entière déposée sous le numéro DSM 15693, surnageant liquide, surnageant concentré et surnageant séché ou lyophilisé obtenu à partir de la souche de *L. reuteri* DAN080 déposée sous le numéro de culture DSM 15693, et de cultures bactériennes mixtes comprenant la souche L. *reuteri* DAN080 déposée sous le numéro DSM 15693, à utiliser dans une méthode pour améliorer l'activité du système immunitaire dans la prophylaxie ou le traitement des infections en augmentant l'activité du lysozyme dans un organisme d'un vertébré, en particulier humain, autre mammifère ou oiseau, le vertébré ayant besoin d'un tel traitement.

6. Utilisation de la préparation telle que définie selon les revendications 4-5, pour la fabrication d'une composition pour la prophylaxie ou le traitement des infections en augmentant l'activité du lysozyme dans un organisme d'un vertébré, en particulier humain, autre mammifère ou oiseau, le vertébré ayant besoin de un tel traitement.

7. Utilisation selon la revendication 3 ou 6, dans laquelle la préparation est destinée à être administrée en une quantité efficace et à un taux approprié, indispensable pour obtenir l'effet recherché.

8. Utilisation selon les revendications 7, dans laquelle la préparation est une composition pharmaceutique, comprenant éventuellement d'autres substances biologiquement actives et vitamines D et E, notamment sous forme de nanoparticules, ainsi que des supports et / ou additifs pharmaceutiquement acceptables.

9. Utilisation selon la revendication 8, dans laquelle la composition pharmaceutique est sous forme solide, de préférence sous forme de comprimé ou de capsule et divisée en doses uniques comprenant une quantité efficace de ladite préparation, allant de 0,001 à 0,2 g / kg de poids corporel par jour.

10. Utilisation selon la revendication 8, dans laquelle la composition pharmaceutique est sous forme liquide, divisée en doses uniques comprenant une quantité efficace de ladite préparation, allant de 0,001 à 0,2 g / kg de poids corporel par jour, notamment en ampoule.

11. Utilisation selon les revendications 3, 6-7, dans laquelle la composition est un complément alimentaire, un aliment sous forme solide et / ou une boisson, comprenant éventuellement d'autres substances biologiquement actives et des vitamines D et E, notamment sous forme de nanoparticules.

12. Utilisation selon les revendications 11, dans laquelle la quantité efficace est comprise entre 0,001 et 0,2 g / kg de poids corporel par jour.

13. Utilisation selon les revendications 3, 6-7, dans laquelle la composition est un aliment ou un additif à un aliment sous forme solide et / ou une boisson, comprenant éventuellement d'autres substances biologiquement actives et des vitamines D et E, notamment sous forme de nanoparticules.

14. Utilisation selon les revendications 13, dans laquelle la quantité efficace varie de 0,001 à 0,2 g / kg de poids corporel par jour.
